# EUROPEAN PATENT APPLICATION

(11) **EP 2 453 243 A1**
(43) Date of publication of application: **16.05.2012**
(21) Application number: 10425348.9
(22) Date of filing: 11.11.2010
(51) Int. Cl.: G01N 33/574, G01N 33/58

(54) **Method for the diagnosis and/or follow up of the evolution of a tumor**

(71) Applicant: Cosmo S.p.A., 20020 Lainata (MI) (IT)
(72) Inventor: Moro, Luigi, 20020 Lainate (MI) (IT); Gerloni, Mara, 20020 Lainate (MI) (IT)
(74) Representative: Pistolesi, Roberto

(57) **Abstract**

A method is disclosed for the diagnosis and/or the follow up of the evolution of cancer by analysis and quantification in the blood/plasma of subject's T cells specific for tumor associated antigens. This is achieved by incubating the peripheral blood of the subjects/patients together with a peptide/major histocompatibility complex (MHC) tetramer that allows the detection and quantization of these specific T cells. The percentage of the T cells in the blood is compared with a baseline range obtained from pre-established healthy subjects' values to determine if the analyzed blood belongs to cancer patients or to healthy control subjects.

SEQUENCE LISTING

<110>  Cosmo Spa

<120>  Method for the diagnosis and/or follow up of the evolution of a
       tumor

<130>  10B440

<160>  4

<170>  PatentIn version 3.3

<210>  1
<211>  9
<212>  PRT
<213>  Homo sapiens

<400>  1

Tyr Met Asp Gly Thr Met Ser Gln Val
1               5


<210>  2
<211>  10
<212>  PRT
<213>  Homo sapiens

<400>  2

Glu Leu Ala Gly Ile Gly Ile Leu Thr Val
1               5                   10


<210>  3
<211>  9
<212>  PRT
<213>  Homo sapiens

<400>  3

Ile Leu Ala Lys Phe Leu His Trp Leu
1               5


<210>  4
<211>  9
<212>  PRT
<213>  Homo sapiens

<400>  4

Tyr Leu Phe Phe Tyr Arg Lys Ser Val
1               5

## Description

This invention is in the field of diagnosis and prognosis of mammalian cancers, preferably human and it relates to a method for the diagnosis and/or the follow up of the evolution of cancer by analysis and quantification in the blood/plasma of subject's T cells specific for tumor associated antigens. This is achieved by incubating the peripheral blood of the subjects/patients together with a peptide/MHC tetramer that allows the detection and quantization of these specific T cells. The percentage of the T cells in the blood is compared with a baseline range obtained from pre-established healthy subjects' values to determine if the analyzed blood belongs to a cancer patient or to a healthy subject. More specifically, this invention relates to levels of CD8 T cells specific for telomerase reverse trascriptase peptides presented in the context of a major histocompatibility complex (MHC) molecule.

### Background of the invention

Telomerase is an enzyme consisting of multiple proteins. It is required to maintain the length of the ends of chromosomes (telomere) and plays a role in determining how many times a cell can divide and replicate. Human telomerase reverse transcriptase (hTERT) is one component of the telomerase enzyme complex and is highly expressed in greater than 85% of human cancer including leukemia but is absent or low in differentiated healthy adult cells.

Like all proteins, hTERT is broken down inside cells into small protein fragments called peptides. These peptides can bind to another protein called MHC and these peptide/MHC complexes are displayed on the surface of cells. Cells displaying these complexes are recognized by the immune system. As part of the immune system, specific T cells recognize these peptide/MHC complexes as either self or foreign antigens. Recognition of these complexes is so specific that only one type of T cell (clone) can recognize a certain peptide/MHC structure. This is the reason for the existence of more than 10 million different T cell clones in a normal human adult.

In healthy persons, there are T cell clones that recognize foreign antigens. These T cells alert and cooperate with other cells of the immune system to react in order to eliminate the cells displaying these foreign antigens. On the other hand, in healthy persons, a self antigen does not lead to an immune reaction because there are little or no T cell clones that can recognize these self antigens. About 26 peptides derived from hTERT have been tested for their ability to elicit immune responses against tumors (Liu et al., 2010, Biochimica et Biophysica Acta 1805:35-42).

The MHC in humans is called HLA (human leukocyte antigen). There are 6 major HLAs in humans: A, B, C, DR, DP, and DQ. Diversity of HLA in the human population is so large that the chance of two unrelated individuals having identical HLA molecules is very low. The 26 identified peptides derived from hTERT bind to six different HLA subtypes expressed in humans. These 6 subtypes are: HLA-A1, -A2, - A3, -A24, -B7, and -DR (reviewed in Liu et al, 2010). More than 75% of persons can be considered for a diagnostic test involving hTERT since the prevalence of these six HLA subtypes in human is in this range.

T cells play an important role in tumor shrinkage or elimination in most animal tumor models. This has spawned a number of immunotherapeutic clinical trials that hope to boost immunity against human tumors. As a way to monitor immune responses against vaccines used in these trials, the number of these T cells specific to the tumor antigens are often measured in the subject's blood. The present invention describes a method of diagnosis and/or follow up of the evolution of most types of cancer, for instance after a chemotherapy or after an operation.

It is known that diagnosis and follow up of the evolution of cancer are done, besides direct observation of the tumors, by biopsy analysis or in the case of some malignancies by analyzing specific tumor markers, generally constituted by antigens, appearing in some body fluids.

Sometimes, conventional methods of diagnosis are not very satisfactory, an early detection isn't easily possible in most cases, and there are a number of disadvantages associated with most forms of cancer screening. These include a high rate of false positive tests, frequent false negative tests and the enormous public health costs involved if the test is complicated. Recently, the development of conventional protein tumor markers, such as carcinoembryonic antigen (CEA) and alpha-fetoprotein (AFP), along with the widely used prostate specific antigen (PSA) was driven largely by the introduction of new methods for quantifying small amounts of circulating proteins. However, sensitivity and specificity shortcomings with these assays still remain to be overcome.

### Description of the invention

The aim of this invention consists therefore in providing an *in vitro* method of diagnosis and/or follow up of the evolution of most types of cancer which would be, on one hand, more precise and trustworthy and, on the other hand easier to perform without implying an invasive test for the patient.

The present inventors have in fact found that, in the blood of persons with cancer, the number of T cells specific to hTERT peptide/MHC is different from healthy subjects.

The present inventors have therefore developed a tumor detection assay in plasma/serum comparing the number of tumor antigen-specific T cells present in the plasma/serum of cancer patients and healthy subjects. The method of the invention is particularly suitable for the diagnosis and/or follow up of the evolution of tumors over-expressing hTERT. In particular, it is suitable for the diagnosis and/or follow up of the evolution of melanoma, preferably a stage I and/or II and/or III and/or IV melanoma.

The method according to the invention comprises the following steps:
(a) detecting and determining the amount of telomerase-specific T cells in the blood of the test mammalian;
(b) detecting and determining the amount of telomerase-specific T cells in the blood of at least a healthy mammalian;
(c) comparing the amount of telomerase-specific T cells determined in step (a) to that determined in step (b).

Both the test mammalian and the healthy mammalian used for the comparison are preferably human beings. The comparison may be carried out over more than one healthy mammalians; in that case the number average amount of telomerase-specific T cells in their blood is determined in step (b).

According to an embodiment of the invention, the peripheral blood of the mammalian is preferably used. According to another embodiment, the blood is purified before detecting and determining the amount of telomerase-specific T cells; preferably, it is purified by density gradient centrifugation.

The amount of telomerase-specific T cells is preferably expressed as the percentage of telomerase-specific T cells that are above the baseline level as defined by a negative control. The telomerase-specific T cells are preferably CD8 telomerase-specific T cells. According to a preferred embodiment of the invention, the telomerase-specific T cells are detected and their amount determined by staining the blood with a peptide/MHC tetramer and subjecting the thus-stained blood to flow cytometry.

A flow cytometer is an instrument that measures properties of single cells. In a flow cytometer, cells in a suspension of fluid pass through a beam of laser light. Each cell is illuminated one at a time. The way a cell scatters light gives information on the size and smoothness or roughness of its surface. More information about the cell can be obtained if the cell has been tagged in the laboratory with antibodies that are chemically linked to a fluorescent molecule. The brightness or intensity of the fluorescence detected is proportional to the number of fluorescent molecules the cell was able to bind. In this way, multiple properties of each single cell that passes through the laser beam can be detected in a quantitative manner. The flow cytometer can rapidly screen large numbers of cells per minute and the information obtained can help diagnose and classify a variety of diseases. A major clinical application of the flow cytometer is in evaluation of immune status, particularly in quantitation of CD4 positive T cells in HIV positive patients and immunophenotyping of blood diseases. In the application for monitoring HIV patients, fluorescently labeled antibodies to markers on a particular type of T cell expressing the proteins, CD4 and CD3, are used. There are many commercially available flow cytometers.

In order to achieve a better comparison between the percentage of the T cells in the blood of the test mammalian with the baseline range obtained from the healthy mammalians, the analyses of data obtained from the flow cytometer exclude non T cells. This is performed after samples have been run through the flow cytometer. Data analyses involve defining regions around known characteristics of T cells such as cell size, granularity and expression of CD3. More specifically, the combination of forward scatter, side scatter, and brightness of the CD3 parameters is intrinsic to T cells. This T cell signature allows for the exclusion of non T cells such as red blood cells, granulocytes, monocytes and/or macrophages, during data analysis.

In addition to antibodies, other reagents that bind only to a specific entity on the cell surface are used to identify other interesting cell properties. A well established reagent that is used to detect T cells specific for a particular peptide antigen/MHC complex is the soluble peptide/MHC tetramer (Savage et al., 1999, Immunity 10:485-492; Yee et al., 1999, J. Immunol. 162:2227-2234; both herein incorporated by reference).

Each tetramer consists of an MHC protein molecule and peptide complex that is tetramerized to create a moiety consisting of 4 peptide/MHC complexes coupled to a fluorescent molecule. Peptide/MHC tetramers bind with high specificity to their cognate T cell receptors (TCR). For example, 2 peptides derived from the same protein (pep1 and pep2) presented by MHC Class I molecule, HLA-A2, will only bind to TCR1 and TCR2, respectively, and will not cross react with each other. In this way, soluble peptide/MHC tetramers are used to quantitate the percentage of specific CD4 or CD8 T cells expressing a particular type TCR. (US Patent Nos. 5,635,363; 5,723,584; 5,874,239; 5,932,433; and 6,265,552. French Application No. FR 9911133; all herein incorporated by reference).

Consequently, the object of the present invention, reaching the above-mentioned aim, is consisting of a method for the diagnosis or the follow up of the evolution of cancers which comprises measuring the percentage of tumor antigen-specific T cells present in the plasma/serum using fluorescently conjugated peptide/MHC tetramers. The difference in the percentage of these T cells in healthy subjects versus cancer patients is significant enough to distinguish between these two different groups.

In the present invention, two peptides derived from hTERT have been chosen as pilot epitopes. However, the present invention is valid for all epitopes of hTERT. To illustrate the present invention, the peptides p540 and pY572 were used. Both of these peptides bind to the MHC class I molecule subtype, HLA-A0201. The p540 peptide is a naturally derived peptide from hTERT and has the following 9 amino acid sequence: ILAKFLHWL. The pY572 peptide is a variant of the naturally derived p572 peptide from hTERT. P572 has the following sequence: RLFFYRKSV. The pY572 peptide is different only in the first amino acid and has the following sequence: YLFFYRKSV. The variant pY572 peptide was chosen due to its higher binding strength to HLA-A0201 than the wild type p572 peptide. (Vonderheide et al., 1999 Immunity 10:673-679; Scardino et al., 2002 J Immunol 168:5900-5906; both herein incorporated by reference). To sum up, the present inventors have shown a different amount of p540 or pY572-specific CD8 T cells in the plasma of patients suffering from melanoma compared to the amount in the blood of healthy subjects. More precisely, the method of diagnosis according to the invention preferably consists of drawing blood from the patient or subject arm then purification to obtain an enriched population of peripheral blood monocytes (PBMC). In the case of blood withdrawn via apheresis, no purification is required since this method of blood withdrawal already enriches for PBMC. Purified blood is then incubated with fluorescent labeled antibodies and tetramer to enumerate the specific T cells by flow cytometry and the data is analyzed using well defined regions around the relevant cell populations. This shall be done in a comparative manner between the plasma or serum of a person suspected of malignancy and the plasma or serum of a healthy person or of a control suffering from a non-malignant disease.

Consequently, according to a preferred embodiment of the present invention, the peptide/MHC tetramer may contain the hTERT peptide epitope p540 conjugated to HLA-A2 or the hTERT peptide epitope pY572 conjugated to HLA-A2.

According to another embodiment of the invention, the peptide/MHC tetramer containing the hTERT peptide epitope p540 conjugated to HLA-A2 and the peptide/MHC tetramer containing the hTERT peptide epitope pY572 conjugated to HLA-A2 are used simultaneously.

Before being stained with the peptide/MHC tetramer, the blood of both test and healthy mammalians is tested for HLA type, preferably for HLA-A2 type, more preferably for HLA-A0201 type.

According to other preferred embodiments, a peptide/MHC tetramer containing the tyrosinase peptide epitope ptyrosinase may be used as negative control and/or a peptide/MHC tetramer containing the MART peptide epitope pMART may be used as positive control.

The application of this invention as a blood test requires only approximately from 5 to 20 ml, preferably from 10 to 15 ml, of peripheral circulating blood to be withdrawn; the blood is preferably purified within 24 hours from withdrawal.

In a typical application, the blood sample is then processed as described below:
a) PBMC are stained with antibodies to CD3, CD8 and tetramer specific to the selected antigen or to control antigens.
b) Stained cells are analyzed by flow cytometry. The data is compared to the negative control antigen in order to select the specific tetramer positive CD8 T cell population. These are expressed as the % of the CD8 T cells that are above the baseline level as defined by the negative antigen.
c) In order to determine if the subject is healthy or tumor bearing, the resulting percentage is compared to the established percentage previously determined in healthy people.

The present invention will now be illustrated in a non-limitative manner by the following example related to the diagnosis of some cancers using hTERT peptide/MHC tetramers.

### Example 1

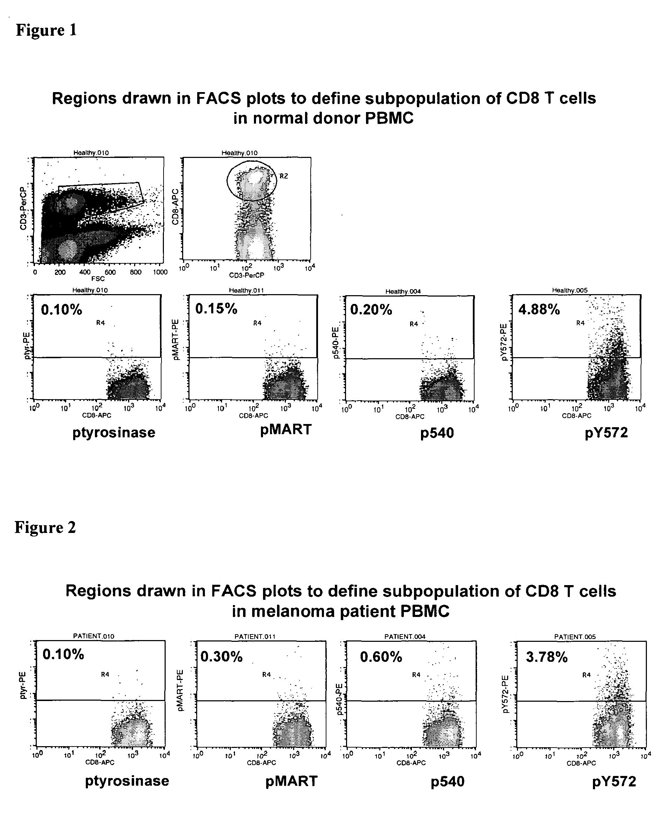

T cells specific for the cancer antigens, pTyr, pMART, and human telomerase p540 and pY572 are quantitated from the subject's blood. The peptide sequences are as follows: pTyr: YMDGTMSQV; pMART: ELAGIGILTV; p540: ILAKFLHWL; pY572: YLFFYRKSV. pTyr was used as a negative control, pMART-1 as the positive control (Pittet MJ et.al., J Exp Med 190:705-716 1999, herein incorporated by reference). PBMC are isolated from 10 ml of whole blood. PBMC are stained with specific antibodies for CD3, CD8, tetramers for tumor associated antigens (pMART, p540, pY572) or pTyr tetramer as control. The samples are then analyzed by flow cytometry. Due to variability of laser power and staining intensity, the analysis region for tetramer positivity was set such that the %pTyr positive CD8 T cells is equivalent to 0.1%. Figures 1 and 2 show the gated regions for healthy human subjects and for the melanoma patients, respectively.

Both figures show typical FACS (fluorescence activated cell sorting) plots, each plot displaying two parameters. In the plot where FSC (forward scatter) is in the x-axis, and CD3-PerCP is in the y-axis, a region is drawn around the area for cells that scatter light that is typical of white blood cells (lymphocytes) and are expressing CD3 on their surface. The gate is labeled R1. The lymphocytes are gated in the CD3 vs. forward scatter plot in order to eliminate red blood cells and other non-T cells. These cells are further evaluated for the presence of CD8 protein on their surface (gate label R2) in order to eliminate CD4 T cells. This subpopulation of cells is evaluated yet again in the plot of CD8-APC in the x-axis and pXXX-PE on the y-axis (gate label R4). The tetramer positive gate is determined from the pTyr vs. CD8 plot and set to 0.1%. The percentage of tetramer+/CD3+/CD8+ cells are obtained for the 3 other tetramers using this negative control gate. The same gating is done for the melanoma patient samples. PerCP, APC, and PE are three different types of fluorophores that emit a different color of light when a blue/green laser beam excites them.

The following table depicts the results of the analysis conducted in the healthy subjects versus melanoma patients with the two different cancer antigens.

**Table 1**

| | Healthy Subjects (N = 36) | Tumor bearing patients (N = 46) |
|---|---|---|
| | average±95% CI | average±95% CI |
| pTyr | 0.10 ± 0.00 | 0.11 ± 0.00 |
| pMART | 0.15 ± 0.02 | 0.29 ± 0.14 |
| p540 | 0.27 ± 0.03 | 0.35 ± 0.06 |
| pY572 | 5.37 ± 1.16 | 2.31 ± 0.73 |

The average percentages of CD8 T cells positive for the tetramers pTyr, pMART-1, p540, and pY572 detected in the blood with the 95% confidence interval shows that normal, healthy subjects and tumor bearing subjects could be easily differentiated by using this test directly on the subject's blood.

The established percentage previously determined in healthy subjects is herein defined as the "reference percentage". Reference percentage can be higher, lower, or the same as patient sample levels. Comparison of a test sample to a reference sample provides an assessment of the hTERT-specific immune response in the test subject. In addition, comparison of a patient's sample levels to reference sample levels can allow a diagnosis of cancer and/or a prognosis of a cancer in a patient having a tumor that includes hTERT-expressing cells. The sample can be obtained from the patient before, during, or after a cancer treatment is administered to the patient.

## Claims

1. A method for the *in vitro* diagnosis and/or follow up of the evolution of a tumor in a test mammalian which comprises the following steps:
(a) detecting and determining the amount of telomerase-specific T cells in the blood of the test mammalian;
(b) detecting and determining the amount of telomerase-specific T cells in the blood of at least a healthy mammalian;
(c) comparing the amount of telomerase-specific T cells determined in step (a) to that determined in step (b).

2. The method according to claim 1, **characterized in that** the average amount of telomerase-specific T cells in the blood of at least two healthy mammalians is determined in step (b).

3. The method according to claim 1, **characterized in that** said test mammalian and healthy mammalian are human beings.

4. The method according to claim 1, **characterized in that** said blood is peripheral blood.

5. The method according to claim 1, **characterized in that** said blood is purified before detecting and determining the amount of telomerase-specific T cells.

6. The method according to claim 5, **characterized in that** said blood is purified by density gradient centrifugation.

7. The method according to claim 1, **characterized in that** the amount of telomerase-specific T cells is expressed as the percentage of telomerase-specific T cells that are above the baseline level as defined by a negative control.

8. The method according to claim 1, **characterized in that** said telomerase-specific T cells are CD8 telomerase-specific T cells.

9. The method according to claim 1, **characterized in that** said telomerase-specific T cells are detected and the amount thereof determined by staining the blood with a peptide/MHC tetramer and subjecting the thus-stained blood to flow cytometry.

10. The method according to claim 9, **characterized in that** said peptide/MHC tetramer contains a hTERT peptide epitope.

11. The method according to claim 10, **characterized in that** said hTERT peptide epitope is conjugated to HLA-A2.

12. The method according to claim 11, **characterized in that** said peptide/MHC tetramer contains the hTERT peptide epitope p540 conjugated to HLA-A2 or the hTERT peptide epitope pY572 conjugated to HLA-A2.

13. The method according to claim 9, **characterized in that** a peptide/MHC tetramer containing the tyrosinase peptide epitope (ptyr) is used as negative control.

14. The method according to claim 9, **characterized in that** a peptide/MHC tetramer containing the MART peptide epitope (pMART) is used as positive control.

15. The method according to claim 9, **characterized in that** the analyses of data obtained from the flow cytometer exclude non T cells.

16. The method according to claim 15, **characterized in that** said non T cells are red blood cells, granulocytes, monocytes and/or macrophages.

17. The method according to claim 9, **characterized in that**, before being stained with the peptide/MHC tetramer, the blood of the mammalian is tested for HLA type, preferably for HLA-A2 type, more preferably for HLA-A0201.

18. The method according to claim 1, **characterized in that** said tumor is a melanoma, preferably a stage I and/or II and/or III and/or IV melanoma.

19. The method according to claim 1, **characterized in that** said tumor is selected from tumors over-expressing hTERT.
